# EUROPÄISCHE PATENTANMELDUNG

(11) **EP 2 098 664 A1**
(43) Veröffentlichungstag der Anmeldung: **09.09.2009**
(21) Anmeldenummer: 08016312.4
(22) Anmeldetag: 16.09.2008
(51) Int. Cl.: E05B 1/00, A61L 2/00

(54) **Handgriff-Überzug, insbesondere für Türgriffe**

(30) Priorität: 08.03.2008 DE 102008013284
(71) Anmelder: Brinkmann, Uwe, 40474 Düsseldorf (DE); Krengel, Klaus-Peter, 44866 Bochum (DE); Schiemenz, Georg, 45356 Essen (DE)
(72) Erfinder: Schiemenz, Georg, 45356 Essen (DE)
(74) Vertreter: Rohmann, Michael

(57) **Zusammenfassung**

Handgriff-Überzug, insbesondere für Türgriffe, wobei der Überzug mit der Maßgabe ausgebildet ist, dass er im übergezogenen Zustand zumindest einen Teil, vorzugsweise den Großteil der Grifffläche des Handgriffes abdeckt. Der Überzug ist fernerhin mit der Maßgabe ausgebildet, dass er im übergezogenen Zustand zumindest bereichsweise formschlüssig an dem zugeordneten Türgriff anliegt. Der Überzug ist aus einem Basismaterial gefertigt und in dem bzw. an dem Basismaterial ist zumindest ein Element mit antibakterieller Wirkung vorgesehen. Das Element mit antibakterieller Wirkung besteht zumindest teilweise aus einem Metall.

## Beschreibung

Die Erfindung betrifft einen Handgriff-Überzug, insbesondere für Türgriffe. Fernerhin betrifft die Erfindung die Verwendung eines Materials, für einen Handgriff-Überzug. Der erfindungsgemäße Handgriff-Überzug kann grundsätzlich über die verschiedensten Handgriffe übergezogen werden, das heißt also über Griffe, die manuell betätigbar sind. Der erfindungsgemäße Handgriff-Überzug wird vorzugsweise für Türgriffe bzw. Türklinken bzw. Türdrücker eingesetzt.

Der Erfindung liegt die Erkenntnis zugrunde, dass über Handgriffe und insbesondere auch über Türgriffe schädliche Bakterien, Mikroorganismen, Keime und Viren übertragen werden. Betroffen sind dabei insbesondere Handgriffe bzw. Türgriffe an Türen in Arztpraxen, Krankenhäusern und dergleichen. Über diese Türgriffe werden Infektionen bzw. Krankheiten in unerwünschter Weise auf eine Vielzahl von Personen übertragen.

Dementsprechend liegt der Erfindung das technische Problem zugrunde den vorstehend beschriebenen Missstand effektiv auszuräumen.

Zur Lösung dieses technischen Problems lehrt die Erfindung einen Handgriff-Überzug, insbesondere für Türgriffe, wobei der Überzug mit der Maßgabe ausgebildet ist, dass er im übergezogenen Zustand zumindest einen Teil, vorzugsweise den Großteil der Grifffläche des Handgriffes abdeckt,
wobei der Überzug fernerhin mit der Maßgabe ausgebildet ist, dass er im übergezogenen Zustand formschlüssig an dem zugeordneten Türgriff anliegt,
wobei der Überzug aus einem Basismaterial gefertigt ist und wobei in dem bzw. an dem Basismaterial zumindest ein Element mit antibakterieller Wirkung vorgesehen ist
und wobei das Element mit antibakterieller Wirkung zumindest teilweise aus einem Metall besteht.

Handgriff meint im Rahmen der Erfindung einen manuell betätigbaren Griff. Türgriff meint im Rahmen der Erfindung auch Türklinke bzw. Türdrücker. Mit antibakterieller Wirkung ist im Rahmen der Erfindung eine Wirkung gegen Bakterien sowie vorzugsweise auch eine Wirkung gegen jegliche Art von schädlichen Keimen, Mikroorganismen und Viren gemeint. - Statt des Begriffes Handgriff-Überzug wird nachfolgend verkürzt auch der Begriff Überzug verwendet.

Nach einer bevorzugten Ausführungsform der Erfindung ist der Überzug schlauchförmig ausgestaltet. Zweckmäßigerweise weist der Überzug dann ein offenes Schlauchende und ein geschlossenes Schlauchende auf. Eine empfohlene Ausführungsform der Erfindung ist dadurch gekennzeichnet, dass der erfindungsgemäße Überzug mit der Maßgabe ausgebildet ist, dass er über einen L-förmigen oder über einen U-fömigen Türgriff überziehbar ist. Zweckmäßigerweise hat der Überzug dann im übergezogenen Zustand auch eine L-Form bzw. eine U-Form. Vorzugsweise läuft der Überzug, insbesondere der schlauchförmige Überzug im übergezogenen Zustand über den Umfang des Türgriffes um.

Es empfiehlt sich, dass der erfindungsgemäße Überzug zumindest 70%, vorzugsweise zumindest 80% und bevorzugt zumindest 90% der Grifffläche des Handgriffes abdeckt. Grifffläche meint im Rahmen der Erfindung insbesondere die Fläche des Handgriffs, die bei einer manuellen Betätigung des Handgriffes üblicherweise von einer Hand erfasst wird bzw. erfasst werden kann.

Es liegt im Rahmen der Erfindung, dass der erfindungsgemäße Überzug mit der Maßgabe ausgebildet, dass er im übergezogenen Zustand zumindest bereichsweise und vorzugsweise vollständig formschlüssig an dem zugeordneten Türgriff anliegt. Nach einer empfohlenen Ausführungsform besteht der erfindungsgemäße Überzug zumindest teilweise aus einem elastischen Material, sodass er sich formschlüssig an den zugeordneten Handgriff bzw. Türgriff anschmiegen kann.

Eine sehr bevorzugte Ausführungsform der Erfindung ist dadurch gekennzeichnet, dass der Überzug als Gestrick oder als Gewebe gefertigt ist. Besonders bevorzugt ist dabei, dass der Überzug als Gestrick gefertigt ist bzw. ein Gestrick ist. Empfohlenermaßen bestehen zumindest 80%, vorzugsweise zumindest 90% und besonders bevorzugt zumindest 95% des Überzugs aus dem Gestrick oder aus dem Gewebe. Es liegt im Rahmen der Erfindung, dass das Basismaterial des Überzuges zu dem Gestrick oder Gewebe verarbeitet ist.

Es empfiehlt sich, dass das Basismaterial zu zumindest 30 Gew.-%, zweckmäßigerweise zu zumindest 35 Gew.-% aus Kunststofffasern besteht. - Es liegt im Rahmen der Erfindung, dass der Überzug 40 bis 80 Gew.-%, vorzugsweise 50 bis 70 Gew.-% und bevorzugt 55 bis 65 Gew.-% Modal aufweist sowie 10 bis 30 Gew.-%, vorzugsweise 15 bis 25 Gew.-% Polyamid aufweist und 6 bis 30 Gew.-%, vorzugsweise 10 bis 25 Gew.-% und bevorzugt 10 bis 20 Gew.-% Elastan aufweist. Es liegt dabei fernerhin im Rahmen der Erfindung, dass das Modal, das Polyamid und das Elastan Bestandteile des Basismaterials des Überzuges sind bzw. das Basismaterial des Überzuges bilden.

Nach besonders bevorzugter Ausführungsform besteht das Element mit der antibakteriellen Wirkung zumindest teilweise, vorzugsweise größtenteils und bevorzugt vollständig aus zumindest einem Metall aus der Gruppe "Silber, Kupfer, Gold, Titan". In besonders vorteilhafter Ausgestaltung der Erfindung besteht das Element mit der antibakteriellen Wirkung zumindest teilweise, vorzugsweise größtenteils und bevorzugt vollständig aus Silber. Größtenteils meint hier, dass zumindest 70 Gew.-%, vorzugsweise zumindest 80 Gew.-% und bevorzugt zumindest 90 Gew.-% des Elementes aus dem Metall bzw. aus dem Silber bestehen. Nach sehr empfohlener Ausführungsform der Erfindung ist das Element mit antibakterieller Wirkung zumindest ein Metallfaden, vorzugsweise zumindest ein Silberfaden, der in den Überzug eingearbeitet ist. Nach sehr bevorzugter Ausführungsvariante ist der Metallfaden bzw. der Silberfaden in das Gestrick aus dem Basismaterial eingewirkt. Zweckmäßigerweise handelt es sich um einen galvanisierten Metallfaden bzw. um einen galvanisierten Silberfaden.

Gemäß empfohlener Ausführungsform der Erfindung weist der Überzug 1 bis 10 Gew.-%, vorzugsweise 2 bis 8 Gew.-% und bevorzugt 4 bis 6 Gew.-% des Elementes bzw. des Materials mit antibakterieller Wirkung auf. Besonders bevorzugt weist der Überzug 5 Gew.-% bzw. in etwa 5 Gew.-% des Elementes bzw. Materials mit Antibakterieller Wirkung auf. Nach einer Ausführungsvariante weist der erfindungsgemäße Überzug 57 bis 61 Gew.-% Modal, 17 bis 22 Gew.% Polyamid, 14 bis 19 Gew.-% Elastan und 4 bis 6 Gew.-% des Metallfadens bzw. Silberfadens auf.

Es liegt im Rahmen der Erfindung, dass zumindest ein Befestigungselement vorgesehen ist, mit dem der Überzug im übergezogenen Zustand an dem Handgriff bzw. Türgriff fixierbar ist. Durch diese Fixierung wird der erfindungsgemäße Überzug bei mänueller Betätigung des Handgriffes gegen ein Abrutschen bzw. Abgleiten von dem Handgriff gesichert. Vorzugsweise ist das Befestigungselement. an dem Handgriff bzw. Türgriff vorgesehen und im übergezogenen Zustand des Überzuges ist der Überzug an diesem Befestigungselement fixierbar. Zweckmäßigerweise läuft das Befestigungselement des Überzuges über zumindest einen Teil des Außenumfangs, vorzugsweise über den gesamten Außenumfang des Türgriffes um. Es empfiehlt sich, dass das Befestigungselement als Klettelement ausgebildet ist. Vorzugsweise läuft dann das Klettelement über den gesamten Außenumfang des Türgriffes um. Es liegt im Rahmen der Erfindung, dass das an dem Türgriff vorgesehene Befestigungselement im übergezogenen Zustand des Überzuges im Bereich des offenen Endes des Überzuges angeordnet ist. Vorzugsweise ist das Befestigungselement mit geringem Abstand vor dem dem Türgriff zugeordneten Schließblech angeordnet. Geringer Abstand meint hier insbesondere 5 bis 20 mm. Aufgrund des erfindungsgemäßen Befestigungselementes kann der Überzug sehr effektiv gegen ein Abrutschen bzw. ein Abgleiten von dem Türgriff gesichert werden.

Die Erfindung betrifft auch die Verwendung eines Materials, das zumindest ein Element mit antibakterieller Wirkung enthält, für einen Handgriff-Überzug, insbesondere für einen Türgriff-Überzug. Soweit hier und vorstehend von einem Element mit antibakterieller Wirkung die Rede ist, meint das nicht zwingend ein Element im chemischen Sinne. Das Element mit antibakterieller Wirkung kann beispielsweise auch eine Verbindung, eine Mischung oder dergleichen sein. Der erfindungsgemäße Handgriff-Überzug wird nach bevorzugter Ausführungsform der Verwendung durch ein Gestrick oder durch ein Gewebe gebildet, in dass das Element mit antibakterietler Wirkung eingearbeitet, vorzugsweise eingewirkt ist. Das Element mit antibakterieller Wirkung besteht dabei zumindest größtenteils, vorzugsweise vollständig aus Silber.

Fernerhin betrifft die Erfindung die Verwendung eines Materials, das zumindest ein Element mit antibakterieller Wirkung enthält, für eine Beschichtung auf einem Handgriff, insbesondere auf einem Türgriff. Es liegt dabei im Rahmen der Erfindung, dass eine Oberflächenbeschichtung des Handgriffes bzw. des Türgriffes das Element mit antibakterieller Wirkung enthält. Weiterhin liegt es im Rahmen der Erfindung, dass das Element mit antibakterieller Wirkung zumindest teilweise aus zumindest einem Metall aus der Gruppe "Silber, Kupfer, Gold, Titan" besteht. Empfohlenermaßen besteht das Element mit antibakterieller Wirkung zumindest teilweise, vorzugsweise größtenteils und bevorzugt vollständig aus Silber bzw. elementarem Silber. Das Metall, vorzugsweise das elementare Silber ist dann in der Beschichtung bzw. Oberflächenbeschichtung des Handgrifles/Türgriffes enthalten. Zweckmäßigerweise ist das Metall bzw. elementare Silber in der Beschichtung bzw. Oberflächenbeschichtung von metallischen Handgriffen, vorzugsweise Türgriffen enthalten. Nach besonders bevorzugter Ausführungsform der erfindungsgemäßen Verwendung ist das Metall, vorzugsweise das elementare Silber durch ein Galvanisierverfahren oder ein Eloxalverfahren auf dem Handgriff, insbesondere auf dem Türgriff aufgebracht. Gemäß dieser bevorzugten Ausführungsform wird die Beschichtung bzw. Oberflächenbeschichtung mit dem Element mit antibakterieller Wirkung also durch ein Galvanisierverfahren oder durch ein Eloxalverfahren aufgebracht. Es liegt somit im Rahmen der Erfindung, dass die Beschichtung bzw. Oberflächenbeschichtung des Handgriffes, vorzugsweise Türgriffes elementares Silber enthält.

Der Erfindung liegt die Erkenntnis zugrunde, dass mit dem erfindungsgemäßen Überzug bzw. mit der erfindungsgemäßen Beschichtung die Übertragung von Keimen bzw. Bakterien und Mikroorganismen sowie Viren sehr effektiv verhindert werden kann. Der Erfindung liegt in diesem Zusammenhang fernerhin die Erkenntnis zugrunde, dass gerade bei einem erfindungsgemäßen Handgriff-Überzug bzw. bei einer erfindungsgemäßen Beschichtung das Element mit antibakterieller Wirkung, insbesondere ein Element aus Silber eine überraschend hohe Wirkung gegenüber Keimen, Bakterien, Mikroorganismen und Viren aufweist. Es ist zu betonen, dass der erfindungsgemäße Überzug bzw. die erfindungsgemäße Beschichtung mit relativ einfachen Mitteln und verhältnismäßig kostengünstig hergestellt werden kann. Insoweit gehen geringe Kosten einher mit einer überraschend effektiven Wirkung.

Nachfolgend wird die Erfindung anhand einer lediglich ein Ausführungsbeispiel darstellenden Zeichnung näher erläutert. Es zeigen in schematischer Darstellung:
- Fig. 1: einen erfindungsgemäßen Handgriff-Überzug und
- Fig. 2: den Gegenstand nach Fig. 1 im übergezogenen Zustand an einem Türgriff.

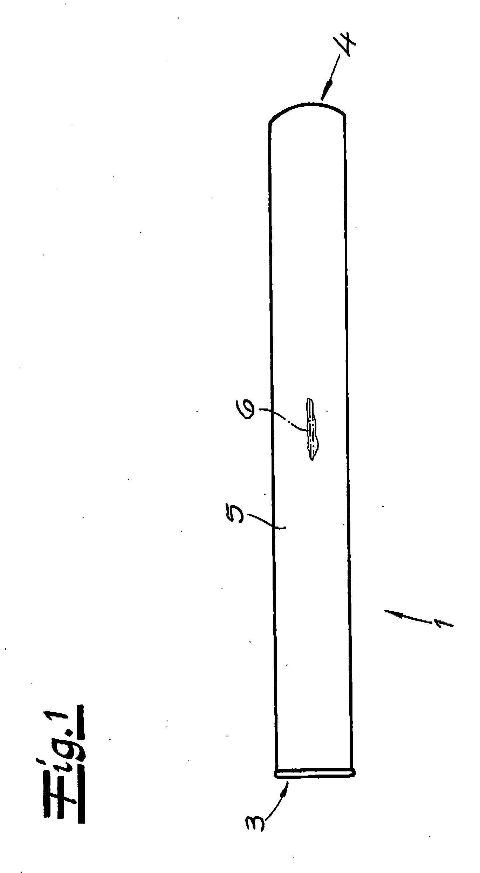

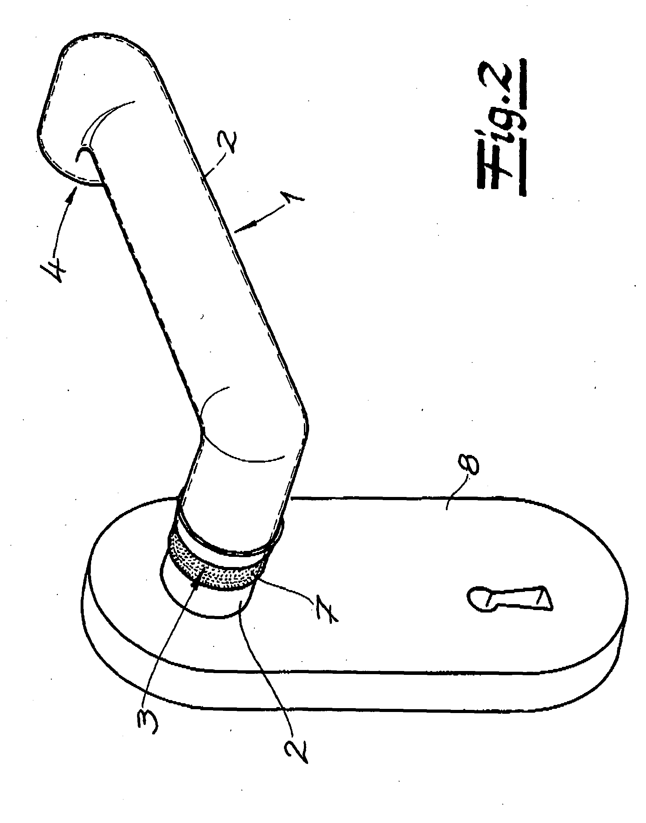

In den Figuren ist ein als Türgriff-Überzug 1 ausgebildeter erfindungsgemäßer Handgriff-Überzug dargestellt. Im Ausführungsbeispiel ist der Überzug 1 schlauchförmig ausgebildet und kann beispielsweise auf einen U-förmigen Türgriff 2 gezogen werden (Fig. 2). Der schlauchförmige Überzug 1 ist an einem Ende 3 offen ausgebildet, und an dem anderen Ende 4 geschlossen ausgebildet (insbesondere Fig. 1). Im übergezogenen Zustand (Fig. 2) deckt der Überzug 1 quasi die gesamte Grifffläche des Türgriffes 2 ab. Der Überzug 1 ist vorzugsweise und im Ausführungsbeispiel mit der Maßgabe ausgebildet, dass er im übergezogenen Zustand formschlüssig an dem Türgriff 2 anliegt (Fig. 2). Nach besonders empfohlener Ausführungsform besteht der Überzug 1 aus einem elastischen Material und kann sich somit auf einfache Weise formschlüssig an den Türgriff 2 anschmiegen.

Der Überzug 1 wird aus einem Basismaterial 5 gefertigt und ist nach besonders bevorzugter Ausführungsform als Gestrick ausgebildet. Das Basismaterial 5 wird dann zu dem Gestrick verarbeitet. Nach empfohlener Ausführungsform enthält das Basismaterial 5 Modal, Polyamid und Elastan. Zweckmäßigerweise wird in das Basismaterial ein Element mit antibakterieller Wirkung eingearbeitet. Bei diesem Element handelt es sich vorzugsweise und im Ausführungsbeispiel um einen Silberfaden 6. Der Überzug 1 besteht vorzugsweise zu 3 bis 7 Gew.% aus diesem Silberfaden 6.

Der Türgriff 2 weist ein als Klettelement 7 ausgebildetes Befestigungselement auf, das bevorzugt und im Ausführungsbeispiel über den gesamten Außenumfang des Türgriffes 2 umläuft. Das Klettelement 7 des Türgriffes 2 ist vorzugsweise und im Ausführungsbeispiel mit Abstand zu dem dem Türgriff 2 zugeordneten Schließblech 8 angeordnet. Der Abstand beträgt zweckmäßigerweise 5 bis 20 mm. lm übergezogenen und befestigten Zustand ist der Überzug 1 im Bereich seines offenen Endes 3 an dem Klettelement 7 fixiert und dadurch wird der Überzug 1 effektiv gegen ein Abrutschen von dem Türgriff 2 gesichert. Insbesondere wenn der Überzug 1 als Gestrick ausgebildet ist, lässt er sich einfach an dem Klettelement 7 fixieren.

Es liegt im Rahmen der Erfindung, dass der erfindungsgemäße Überzug 1 in bestimmten Zeitabständen, beispielsweise in Zeitabständen von 2 Wochen ausgewechselt wird. Hervorzuheben ist, dass der erfindungsgemäße Überzug 1 aufgrund seiner Ausgestaltung einfach und zügig ausgewechselt werden kann. Der Überzug 1 zeichnet sich an einem Handgriff bzw. Türgriff 2 durch eine hervorragende antibakterielle Wirkung aus. Bakterien und dergleichen werden quasi umgehend abgetötet und somit kann auf sehr effektive Weise die Übertragung von Infektionen bzw. Krankheiten über Handgriffe und insbesondere über Türgriffe 2 vermieden werden.

## Patentansprüche

1. Handgriff-Überzug, insbesondere für Türgriffe (2), wobei der Überzug (1) mit der Maßgabe ausgebildet ist, dass er im übergezogenen Zustand zumindest einen Teil, vorzugsweise den Großteil der Grifffläche des Handgriffes abdeckt,
wobei der Überzug fernerhin mit der Maßgabe ausgebildet ist, dass er im übergezogenen Zustand zumindest bereichsweise formschlüssig an dem zugeordneten Türgriff anliegt,
wobei der Überzug (1) aus einem Basismaterial (5) gefertigt ist und wobei in dem bzw. an dem Basismaterial (5) zumindest ein Element mit antibakterieller Wirkung vorgesehen ist
und wobei das Element mit antibakterieller Wirkung zumindest teilweise aus einem Metall besteht.

2. Überzug nach Anspruch 1, wobei der Überzug (1) schlauchförmig ausgestaltet ist.

3. Überzug nach einem der Ansprüche 1 oder 2, wobei der Überzug (1) zumindest 70%, vorzugsweise zumindest 80% und bevorzugt zumindest 90% der Grifffläche des Handgriffes abdeckt.

4. Überzug nach einem der Ansprüche 1 bis 3, wobei der Überzug (1) als Gestrick oder als Gewebe gefertigt ist.

5. Überzug nach einem der Ansprüche 1 bis 4, wobei das Basismaterial (5) zu zumindest 30 Gew.-%, vorzugsweise zu zumindest 35 Gew.-% aus Kunststofffasem besteht.

6. Überzug nach einem der Ansprüche 1 bis 5, wobei der Überzug (1) 40 bis 80 Gew.-%, vorzugsweise 50 bis 70 Gew.-% und bevorzugt 55 bis 65 Gew.% Modal aufweist, sowie 10 bis 30 Gew.-%, vorzugsweise 15 bis 25 Gew.-% Polyamid aufweist und 6 bis 30 Gew.-%, vorzugsweise 10 bis 25 Gew.-% und bevorzugt 10 bis 20 Gew.-% Elastan aufweist.

7. Überzug nach einem der Ansprüche 1 bis 6, wobei das Element mit antibakterieller Wirkung zumindest teilweise aus zumindest einem Metall aus der Gruppe "Silber, Kupfer, Gold, Titan" besteht und vorzugsweise zumindest teilweise aus Silber besteht

8. Überzug nach einem der Ansprüche 1 bis 7, wobei das Element mit antibakterieller Wirkung zumindest ein Metallfäden, vorzugsweise zumindest ein Silberfaden ist, der in den Überzug (1) eingearbeitet ist.

9. Überzug nach einem der Ansprüche 1 bis 8, wobei der Überzug (1) 1 bis 10 Gew.-%, vorzugsweise 2 bis 8 Gew.-% und bevorzugt 4 bis 6 Gew.-% des Elementes bzw. des Materials mit antibakterieller Wirkung aufweist.

10. Überzug nach einem der Ansprüche 1 bis 9, wobei der Türgriff (2) zumindest ein Befestigungselement aufweist, mit dem der Überzug (1) im übergezogenen Zustand an dem Handgriff fixierbar ist.

11. Überzug nach Anspruch 10, wobei das Befestigungselement über zumindest einen Teil des Außenumfangs, vorzugsweise über den gesamten Außenumfang des Türgriffs (2) umläuft.

12. Überzug nach einem der Ansprüche 10 oder 11, wobei das Befestigungselement ein Klettelement (7) ist.

13. Verwendung eines Materials, das zumindest ein Element mit antibakterieller Wirkung enthält, für einen Handgriff-Überzug, insbesondere für einen Türgriff-Überzug.

14. Verwendung eines Materials, das zumindest ein Element mit antibakterieller Wirkung enthält, für eine Beschichtung eines Handgriffes, insbesondere eines Türgriffes.

15. Verwendung nach einem der Ansprüche 13 oder 14, wobei das Element mit antibakterieller Wirkung zumindest teilweise aus zumindest einem Metall aus der Gruppe "Silber, Kupfer, Gold, Titan" besteht und vorzugsweise zumindest teilweise aus Silber besteht.
